(19) Europäisches Patentamt
European Patent Office
Office européen des brevets

(11) **EP 4 250 301 A1**

(12) **EUROPEAN PATENT APPLICATION**

(43) Date of publication:
**27.09.2023 Bulletin 2023/39**

(51) International Patent Classification (IPC):
**G16B 40/20** (2019.01)   **G16B 45/00** (2019.01)
**G16H 50/20** (2018.01)   G16B 20/10 (2019.01)
G16B 20/50 (2019.01)   G16B 25/10 (2019.01)

(21) Application number: **23163839.6**

(22) Date of filing: **23.03.2023**

(52) Cooperative Patent Classification (CPC):
**G16B 40/20; G16B 45/00; G16H 50/20;**
G16B 20/10; G16B 20/50; G16B 25/10

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **24.03.2022   IT 202200005861**

(71) Applicant: **Aizoon S.r.l.**
**10146 Torino (TO) (IT)**

(72) Inventors:
• **MANGANARO, Lorenzo**
**10146 Torino (TO) (IT)**

• **SABBATINI, Gianmarco**
**10146 Torino (TO) (IT)**
• **BIANCO, Selene**
**10146 Torino (TO) (IT)**
• **CIPOLLINI, Francesca**
**10146 Torino (TO) (IT)**
• **COLOMBI, Davide**
**10146 Torino (TO) (IT)**
• **Vattakunnel, Shaji**
**10146 Torino (TO) (IT)**
• **FALCO, Paolo**
**10146 Torino (TO) (IT)**

(74) Representative: **Meindl, Tassilo**
**Buzzi, Notaro & Antonielli d'Oulx S.p.A.**
**Corso Vittorio Emanuele II, 6**
**10123 Torino (IT)**

(54) **METHOD FOR ESTIMATING A VARIABLE OF INTEREST ASSOCIATED TO A GIVEN DISEASE AS A FUNCTION OF A PLURALITY OF DIFFERENT OMICS DATA, CORRESPONDING DEVICE, AND COMPUTER PROGRAM PRODUCT**

(57)     Described herein are solutions for estimating a variable of interest (v) associated to a given disease as a function of omics data (300) of a patient (P).

During a training step (1100), a computer receives (1102) a first dataset of omics data (200'$_1$) and a second dataset of omics data (200'$_2$), wherein each dataset of omics data (200'$_1$, 200'$_1$) comprises the values of a respective plurality of variables that refer to the same genes for each reference patient (PR) of a plurality of reference patients (PR1, PR2, PR3). Next, the computer generates (1104-1110) a multi-layer network (204) comprising a first layer (200'$_1$) and a second layer (200'$_2$) with respective intra-omics connections (W$_1$, W$_2$) and inter-omics connections (W$_{12}$), and prunes (1108) non-salient intra-omics connections and inter-omics connections of the multi-layer network (204) by means of a backboning method. Next, the computer identifies (1112) a plurality of communities (206) of the multi-layer network (204), and determines (1114), via a feature-extraction method, for each community (206) one or more respective features (F). Finally, the computer generates (1116) a training dataset (210), obtaining for each reference patient (PR) a respective value of the variable of interest (v) and calculating for each reference patient (PR) the respective values of the features (F), and the computer trains (1118) a classifier configured for estimating the value (v') of the variable of interest (v) using the training dataset (210).

Consequently, during an estimation step (1200), the computer receives (1102) the values (300) of the variables for a patient (P), calculates the values (302) of the features (F), and estimates (1204), by means of the trained classifier, the value (v') of the variable of interest (v).

FIG. 10

## Description

Technical field

Various embodiments of the present disclosure regard solutions for estimating a variable of interest associated to a given disease as a function of a plurality of different omics data of a patient.

Background

It is deemed that gene profiles can be linked to the risk of developing a given disease and/or to the prognosis of evolution of the disease, such as a particular type of cancer or neoplasm. For instance, the prognosis is frequently quantified through a quantity that indicates the time of disease-free survival (DFS) after a particular treatment.

With the invention and recent reduction in cost of next-generation sequencing (NGS) technology, a large amount of *omics* data has become progressively available for biocomputational analyses. In particular, NGS technology is an *in vitro* process of analysis that comprises sequencing in parallel and that enables sequencing of large genomes over a very restricted time. The term "omics" refers to data that identify genomics, transcriptomics, proteomics, metabolomics, and/or metagenomics.

This has in turn increased the interest in the development of automatic learning, i.e., machine learning (ML), models that are able to decode the correlations between different gene profiles (also referred to as "omics profiles"), for example with reference to gene mutation (differences between individuals encoded in different DNA sequences), expression (differences between individuals and tissues deriving from the effective process used for producing proteins, encoded in the RNA), and copy numbers (differences between individuals and tissues deriving from a different copy numbers of a given gene). For instance, in this context, European patents EP 1 977 237 B1, EP 2 392 678 B1, EP 2 836 837 B1, EP 3 237 638 B1 or EP 2 700 038 B1 may be cited.

For instance, to estimate the risk of developing a given disease or the disease-free survival time of a patient, the machine-learning model may comprise a parameterized mathematical function, such as an artificial neural network, configured for estimating a quantity of interest that corresponds, respectively, to the risk of developing the disease or the disease-free survival time, as a function of the omics data obtained for a given patient. In particular, by acquiring a training dataset that comprises the omics data of a plurality of patients and the respective information as to whether the patient has developed the disease, or the respective disease-free survival time of each patient, a training algorithm can modify, typically through an iterative process, the parameters of the mathematical function in such a way as to reduce the difference between the estimations of the quantity of interest and the respective data of the dataset. Consequently, once the learning model has been trained, the mathematical function can provide an estimate of the quantity of interest, i.e., the risk of developing the disease or the disease-free survival time as a function of the respective omics data of a patient.

One of the main problems in this field is that the training dataset typically includes a limited number of samples, especially if compared to the enormous number of features that need to be analyzed for an effective prognosis. In fact, clinical trials typically involve from a few hundreds up to 1500-2000 patients, whereas NGS data may potentially provide as many features as are the human genes: approximately 20 000 in the case of transcriptome if the analysis is limited to the genes that encode proteins, and a factor of two to three times more if also the non-encoding genes are considered. From the standpoint of automatic learning, this problem of dimensionality (generally known as "curse of dimensionality") prevents the models from learning, leading to an over-adaptation with respect to the training dataset and to the loss of the capacity of generalization since there are not enough samples for extrapolating a general behaviour.

Summary

Various embodiments of the present disclosure hence provide new solutions that are able to handle the aforesaid problem of dimensionality by extracting mainly the relevant information from the data, thus reducing the number of features and solving the problem of dimensionality.

According to one or more embodiments, the above object is achieved through a method having the distinctive elements set forth specifically in the ensuing claims. The embodiments moreover regard a corresponding device, as well as a corresponding computer program product that can be loaded into the memory of at least one computer and comprises portions of software code for executing the steps of the method when the product is run on a computer. As used herein, reference to such a computer program product is understood as being equivalent to reference to a computer-readable means containing instructions for controlling a processing system in order to co-ordinate execution of the method. Reference to "at least one computer" is clearly intended to highlight the possibility of the present description being implemented in a distributed/modular way.

The claims form an integral part of the technical teaching of the description provided herein.

As mentioned previously, various embodiments of the present disclosure relate to solutions for estimating the value of a variable of interest associated to a given disease, such as a neoplasm or cancer, such as a non-small-cell lung cancer, as a function of omics data of a patient. For instance, the variable of interest may indicate whether the respective patient has developed the disease, the seriousness of the disease that the respective patient has developed, or a disease-free survival time of the respective patient.

In various embodiments, during a training step, a computer receives a first dataset of omics data and a second dataset of omics data, where each dataset of omics data comprises the values of a respective plurality of variables that refer to the same genes for each reference patient of a plurality of reference patients. For instance, the datasets of omics data may be chosen from among: a dataset of transcriptomic data, where each variable corresponds to the gene expression of a particular gene, for example expressed in transcripts per million; a dataset of copy number variation data, where each variable corresponds to the copy number variations of a particular gene; and a dataset of mutation data, where each variable corresponds to the mutation data of a particular gene.

In various embodiments, the computer then generates a multi-layer network comprising a first layer and a second layer. In particular, for this purpose, the computer associates to each variable of the first dataset of omics data a respective node in the first layer and to each variable of the second dataset of omics data a respective node in the second layer. Next, the computer generates intra-omics connections, calculating for each pair of nodes of the first layer and each pair of nodes of the second layer a respective value of similarity as a function of the data of the respective variables associated to the two nodes, and calculating for each pair of nodes of the first layer and each pair of nodes of the second layer a respective weight associated to the connection between the respective nodes as a function of the value of similarity of the respective pair of nodes. For instance, in various embodiments, in the case where the respective dataset of omics data comprises transcriptomic data or copy number variation data, the computer calculates the respective similarity values via the biweight-midcorrelation metric. Instead, in the case where the respective dataset of omics data comprises mutation data, the computer calculates the respective similarity values via the normalized mutual information. Moreover, the computer generates inter-omics connections, calculating for each pair of nodes between the first layer and the second layer a respective value of similarity as a function of the data of the respective variables associated to the two nodes, and calculating for each pair of nodes between the first layer and the second layer a respective weight associated to the connection between the respective nodes as a function of the value of similarity of the respective pair of nodes. For instance, in various embodiments, in the case where the respective datasets of omics data comprise transcriptomic data and copy number variation data, the computer calculates the respective similarity values via the biweight-midcorrelation metric. Instead, in the case where the respective datasets of omics data comprise other data, the computer can calculate the respective similarity values via the point-biserial correlation.

In various embodiments, the computer then removes non-salient intra-omics connections and inter-omics connections of the multi-layer network by applying to the weights associated to the intra-omics connections between the nodes of the first layer, to the weights associated to the intra-omics connections between the nodes of the second layer, and to the weights associated to the inter-omics connections between the nodes of the first layer and the second layer, a backboning method, such as the NC (Noise-Corrected) method.

In various embodiments, the computer can use also a third dataset of omics data. In this case, the computer can then associate to each variable of the third dataset of omics data a respective node in a third layer and generate intra-omics connections, calculating, for each pair of nodes of the third layer, a respective value of similarity as a function of the data of the respective variables associated to the two nodes, and calculating, for each pair of nodes of the third layer, a respective weight associated to the connection between the respective nodes as a function of the value of similarity of the respective pair of nodes. Moreover, to generate inter-omics connections, the computer can calculate, for each pair of nodes between the first layer and the third layer and each pair of nodes between the second layer and the third layer, a respective value of similarity as a function of the data of the respective variables associated to the two nodes, and calculate, for each pair of nodes between the first layer and the third layer and each pair of nodes between the second layer and the third layer, a respective weight associated to the connection between the respective nodes as a function of the value of similarity of the respective pair of nodes. Likewise, the computer can then remove the non-salient intra-omics connections and inter-omics connections of the multi-layer network by applying also to the weights associated to the intra-omics connections between the nodes of the third layer, to the weights associated to the inter-omics connections between the nodes of the first layer and the nodes of the third layer, and to the weights associated to the inter-omics connections between the nodes of the second layer and the nodes of the third layer, the backboning method.

In various embodiments, the computer then identifies a plurality of communities/sub-networks of the multi-layer network, for example by performing a plurality of executions of the Infomap method. Next, the computer determines, via a feature-extraction method, such as the UMAP (Uniform Manifold Approximation and Projection) method, for each community one or more respective features as a function of the values of the variables associated to the nodes that belong to the respective community, and stores the mapping rules used for generating the one or more features as a function of the values of the variables.

In various embodiments, the computer then generates a training dataset, by obtaining, for each reference patient, a respective value of the variable of interest and calculating, for each reference patient, the respective values of the features associated to the communities as a function of the respective values of the variables of the reference patient using the mapping rules. Finally, the computer uses the training dataset for training a classifier configured for estimating the value of the variable of interest as a function of the values of the features.

Consequently, during an estimation step, the computer receives the values of the variables of the datasets of omics data for a further patient, calculates for the patient the values of the features as a function of the respective values of the variables of the patient using the mapping rules, and estimates, by means of the trained classifier, the value of the variable of interest as a function of the values of the features calculated for the patient.

Brief description of the drawings

The embodiments of the present disclosure will now be described with reference to the annexed drawings, which are provided purely by way of non-limiting example and in which:

- Figure 1 shows a flowchart of a method and the corresponding operation of a computer configured for estimating a variable of interest as a function of the omics data of a patient via a learning step and an estimation step;
- Figure 2 shows an example of a processing system capable of implementing the operation of Figure 1;
- Figure 3 shows an embodiment of the learning step of Figure 1;
- Figures 4, 5, 6, 7, 9, 10, 11, 12 show details of the learning step of Figure 3;
- Figure 13 shows an embodiment of the estimation step of Figure 1; and

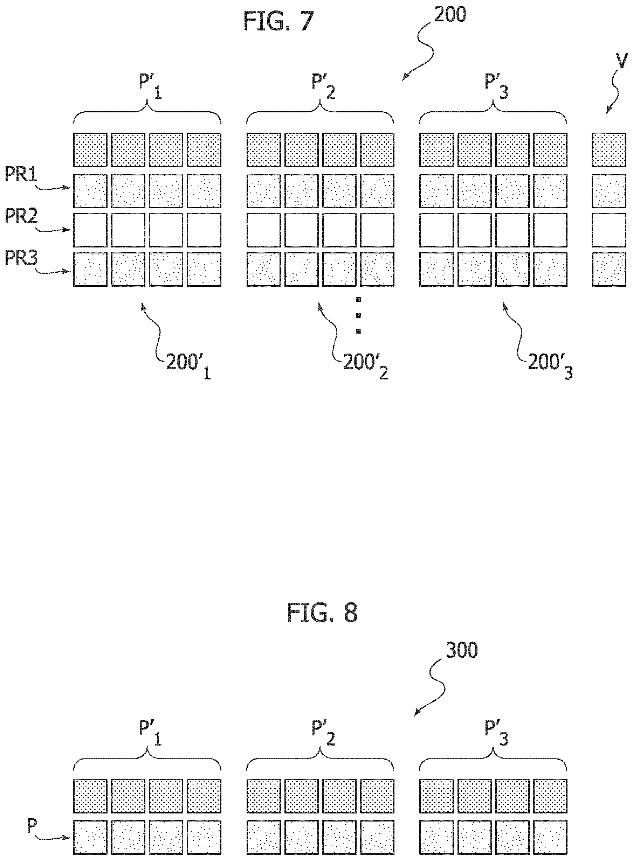

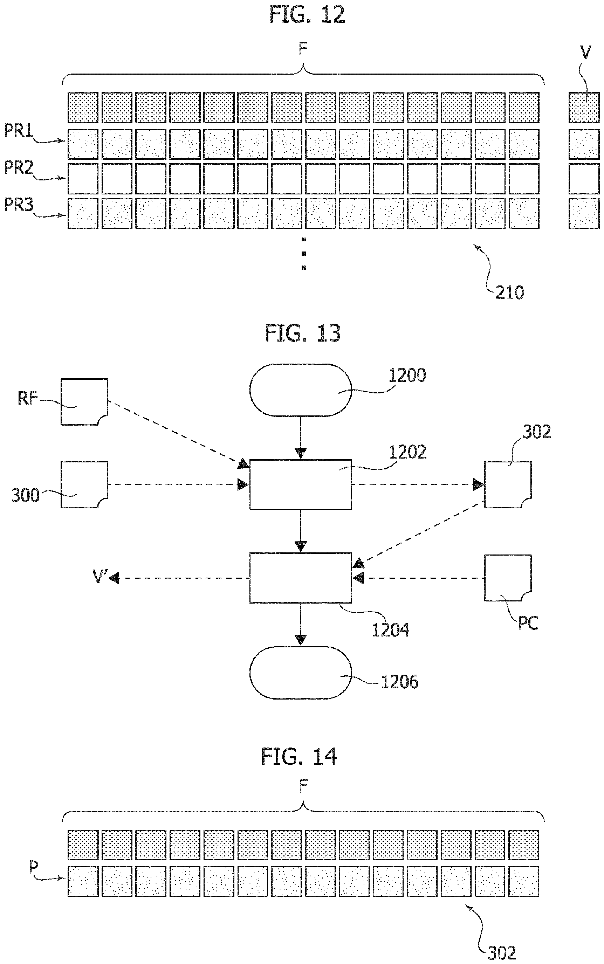

- Figures 8 and 14 show details of the estimation step of Figure 13.

Detailed description

In the ensuing description numerous specific details are provided in order to enable an in-depth understanding of the embodiments. The embodiments may be implemented without one or various specific details, or with other methods, components, materials, etc. In other cases, well-known operations, materials, or structures are not represented or described in detail so that the aspects of the embodiments will not be obscured.

Reference throughout the ensuing description to "an embodiment" or "one embodiment" means that a particular characteristic, distinctive element, or structure described with reference to the embodiment is comprised in at least one embodiment. Thus, use of phrases such as "in an embodiment" or "in one embodiment" in various points of this description do not necessarily refer to one and the same embodiment. Moreover, the details, characteristics, distinctive elements, or structures may be combined in any way in one or more embodiments.

The references used herein are provided merely for convenience and do not define the scope or meaning of the embodiments.

As explained previously, to enable a more accurate prognosis, recently also omics data are taken in consideration, which are processed by means of a machine-learning algorithm. For instance, in various embodiments, the present solution is used for estimating the disease-free survival time for patients affected by non-small cell lung cancer (NSCLC), who have undergone a surgical operation and have obtained complete removal of the neoplasm. However, the solution proposed herein may be used for estimating the disease-free survival time also for other diseases, and in particular for other types of neoplasms. For instance, such an estimate may represent auxiliary information fundamental for oncologists, who can make better-informed decisions, for example vary the frequency of the follow-up checks as a function of the time estimated, for instance increase the frequency of the checks for patients with an estimated time that is short. In general, the solution described herein may be used for estimating also one or more other quantities of interest that are correlated with the omics data of a patient, such as the risk of a patient developing a given disease and/or the risk of a patient developing a serious form of the disease.

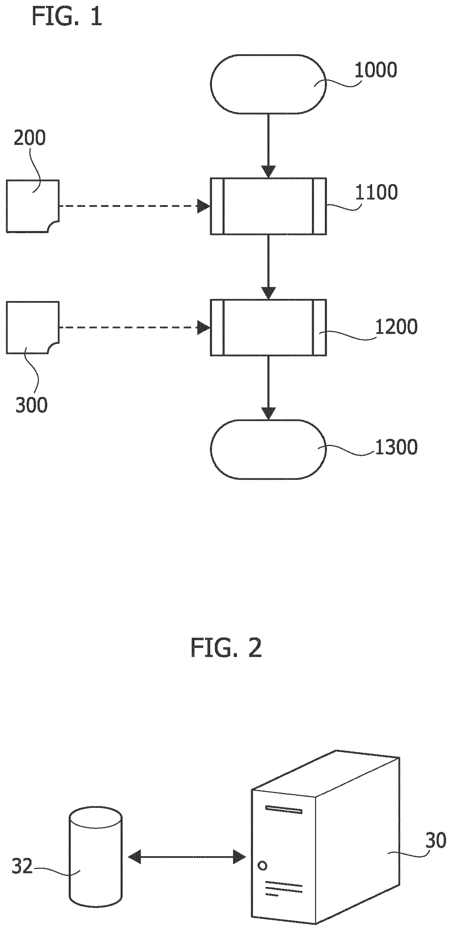

Figure 1 shows an embodiment of operation of a computer 30 configured for estimating a variable of interest for a given patient (output datum) as a function of respective values of a set of parameters (input data).

For instance, as illustrated in Figure 2, the computer 30 can be implemented with any processing system, possibly also in distributed form, and may, for example, comprise a computer, a smartphone or tablet, and/or a remote server. Consequently, operation of the computer 30 can be implemented via software code executed by one or more processors. For instance, in this case, the dataset 200 can be stored in one or more databases 32 managed by the computer 30.

In the embodiment considered, after a starting step 1000, the computer 30 trains, in a step 1100, a machine-learning algorithm using a training dataset 200 that comprises omics data for a plurality of reference patients PR. Consequently, in a step 1200, the computer 30 can use the trained algorithm for estimating the quantity of interest, for example the disease-free survival time, of a patient as a function of the respective omics data 300, and the method terminates in an end step 1300.

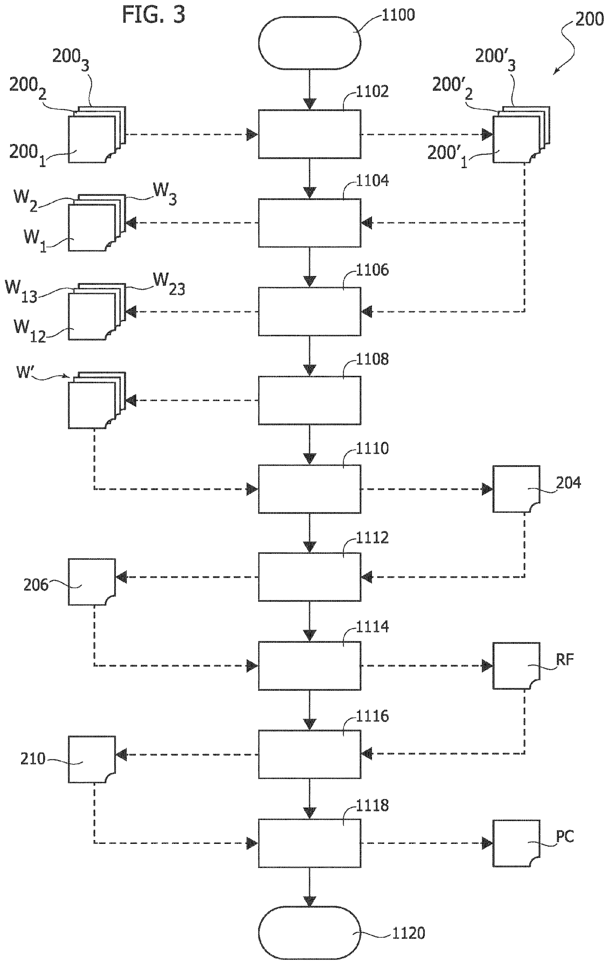

Figure 3 shows a possible embodiment of the training step 1100. Once the straining step 1100 has been started, the computer 30 obtains the training dataset 200.

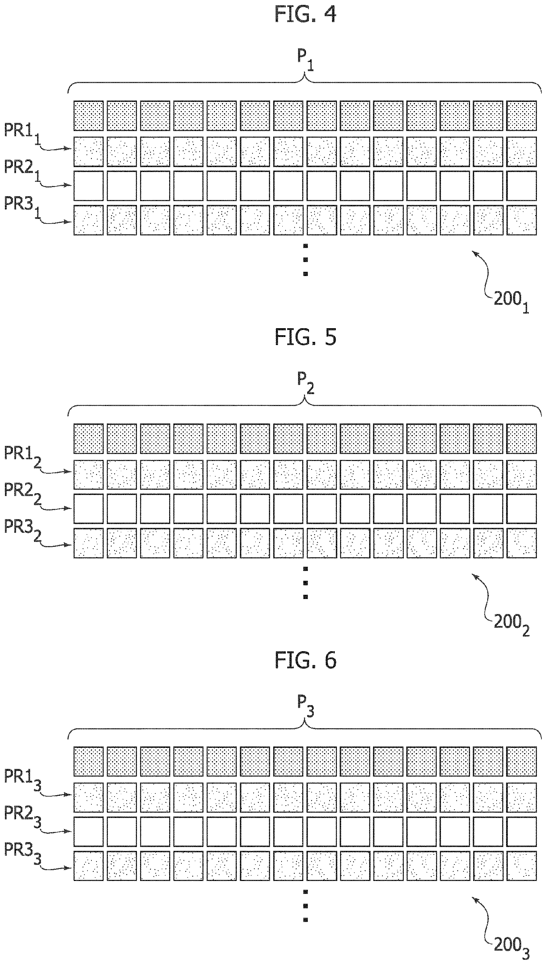

In particular, Figure 4 shows an example of a dataset $200_1$ of NGS transcriptomic data (RNA-seq). In particular, the dataset $200_1$ corresponds to a table, list, or array of data that comprises the values for a number $p_1$ of variables for a number $m_1$ of reference patients PR. In particular, with reference to NGS data, each variable corresponds to the gene expression of a particular gene. For instance, the dataset $200_1$ may correspond to a data array in which each row of the array represents a reference patient $PR_1$ (sample), for example patients $PR1_1$, $PR2_1$, $PR3_1$, etc., and each column represents the gene expression of a particular gene, for example expressed in transcripts per million (TPM).

Figure 5 shows an example of a dataset $200_2$ of copy number variation data. In this case, the dataset $200_2$ corresponds to a table, list, or array of data that comprises the values for a number $p_2$ of variables, where each variable corresponds to the copy number variations of a particular gene, for a number $m_2$ of reference patients $PR_2$, for example patients $PR1_2$, $PR2_2$, $PR3_2$, etc.

Finally, Figure 6 shows an example of a dataset $200_3$ of mutation data. In this case, the dataset $200_3$ corresponds to a table, list, or array of data that comprises the values for a number $p_3$ of variables, where each variable corresponds to the data of mutation of a particular gene, for a number $m_3$ of reference patients $PR_3$, for example patients $PR1_3$, $PR2_3$, $PR3_3$, etc.

For instance, some examples of these data are made available by the *Cancer Genome Atlas* (TCGA) and can be freely downloaded from many sources, such as https://gdac.broadinstitute.org/ or https://www.cbioportal.org/. For instance, with reference to NSCLC, a dataset 200 can be used that comprises the databases TCGA lung adenocarcinomas (LUADs) and/or TCGA lung squamous-cell carcinomas (LUSCs), which currently represent the main histological subtypes of NSCLC.

In general, the datasets $200_1$, $200_2$ and $200_3$ may refer to different sets of patients PR and/or different genes. For instance, with reference to the NSCLC data of the TCGA database, the datasets $200_1$, $200_2$ and $200_3$ comprise the following data:

- dataset $200_1$: $m_1$ = 889 patients and $p_1$ = 16549 genes selected for the mRNA gene expression;
- dataset $200_2$: $m_2$ = 998 patients and $p_2$ = 25128 genes selected for the copy number variations; and
- dataset $200_3$: $m_3$ = 1030 patients and $p_3$ = 18213 genes selected for the mutations.

Consequently, as illustrated in Figure 3, the computer 30 can pre-process, in a step 1102, the datasets $200_1$, $200_2$, and $200_3$, to generate a single dataset 200. For instance, for this purpose, the computer 30 can select only those reference patients PR and genes that are common to all three omics considered $200_1$, $200_2$, and $200_3$. For instance, by selecting these data, the computer can obtain datasets $200'_1$, $200'_2$, and $200'_3$, where each of these datasets has the same numbers m = 847 of patients and p = 15736 of genes.

Consequently, as also illustrated in Figure 7, in the embodiment considered, the dataset 200 comprises the ensemble of the datasets $200'_1$, $200'_2$, and $200'_3$, i.e., for each of the m reference patients PR, for example patients PR1, PR2, PR3, etc., the respective $p'_1$ mRNA gene expressions $200'_1$, the respective $p'_2$ copy number variations $200'_2$, and the respective $p'_3$ mutations $200'_3$, where these parameters refer to the same p genes. Moreover, the training dataset 200 comprises, for each reference patient PR, the value of the variable of interest v, such as:

- the information as to whether the patient has developed the disease;
- data that indicate the seriousness of the disease that the patient has developed, for example if the patient has developed a mild form or a serious form (with hospital admission and/or hospital intervention and/or death of the patient due to the disease); or
- data that identify the respective disease-free survival time, for example data that indicate a period, e.g., a number of days, from the date of the intervention, such as a period that has elapsed up to a relapse or, in the absence of relapse, a period that has elapsed up to the last check, or a period that has elapsed up to the death of the patient.

In general, the step 1102 is purely optional, since the computer 30 could directly receive a similar dataset 200.

Consequently, as illustrated in Figure 8, in various embodiments, the data 300 of a patient P comprise $p'_1$ mRNA gene expressions $300_1$, $p'_2$ copy number variations $300_2$, and $p'_3$ mutations $300_3$, where these data refer to the same p genes. Consequently, the computer 30 should estimate, in step 1200, a respective value v as a function of these data 300. For instance, in the embodiment considered, the computer 30 should determine the correlation of the variable of interest v with $3 \cdot p$ parameters.

In this context, the inventors have noted that network analysis can be a very effective approach for analysing the biological complexity inherent in oncogenic processes. Computational methods based upon networks are typically applied for studying separately biological measurements, such as the omics data $200'_1$, $200'_2$, and $200'_3$, even if these

are not altogether independent.

Instead, to take into consideration the data 200, a multi-network approach should be used. In fact, a multidimensional network is a set of nodes that interact with one another in a number of different dimensions or layers, each of which reflects a distinct type of interaction that connects one and the same pair of nodes. Multidimensional networks have emerged recently as a subject of great interest (e.g., BOCCALETTI, Stefano, et al., "The structure and dynamics of multilayer networks", Physics reports, 2014, 544.1: 1-122; KIVELÄ, Mikko, et al., "Multilayer networks", Journal of complex networks, 2014, 2.3: 203-271) and have produced precious information in multiple fields, amongst which integration of multiple omics in the oncological field (e.g., CANTINI, Laura, et al., "Detection of gene communities in multinetworks reveals cancer drivers", Scientific reports, 2015, 5.1: 1-10; and HIDALGO, Sebastian J. Teran; MA, Shuangge, "Clustering multilayer omics data using MuNCut", BMC genomics, 2018, 19.1: 1-13.).

Consequently, for the dataset 200 considered by way of example, the multidimensional network would have p nodes in three dimensions, where the three dimensions refer, respectively, to the data $p'_1$, $p'_2$, and $p'_3$ that correspond the same p genes. In general, taking into consideration fewer or more datasets of omics data, the number of the layers can vary accordingly. Hence, in the context of omics data, the computer 30 would have to correlate an enormous amount of information, for example deriving from mutations, copy number variations, and data on mRNA gene expression, in a multidimensional network. In fact, as mentioned previously, the number p of the parameters of each network may be easily higher than 5000, 10 000 or even 15 000 genes.

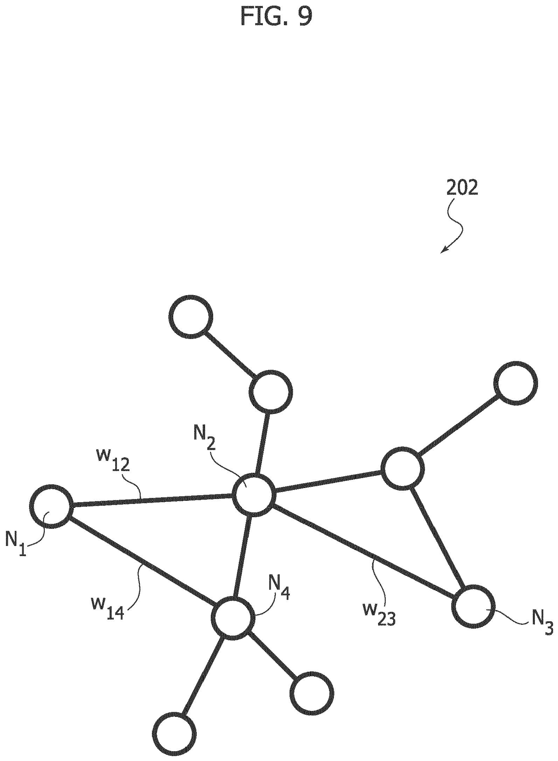

For this purpose, the computer 30 generates for each of the datasets $200'_1$, $200'_2$, and $200'_3$ a respective weighted network 202, i.e., networks $202_1$, $202_2$, and $202_3$. In this case, as also illustrated in Figure 9, each gene of the p genes (i.e., each of the $p'_1$, $p'_2$, or $p'_3$ variables of the respective dataset) corresponds to a node $N_i$ of the network 202, with $1 \leq i \leq p$, and the weights $w_{ij}$ of the edges between two nodes $N_i$ and $N_j$, with $1 \leq j \leq p$ and $j \neq i$, are proportional to the mutual correlation between the two connected genes.

In particular, for this purpose, the computer generates in step 1104 for each dataset $200'_1$, $200'_2$, and $200'_3$ a respective similarity matrix S. This matrix S is a symmetrical square matrix of size p × p, where each element $s_{ij}$ contains a measurement of correlation between the gene $i$ and the gene $j$ (i.e., of the respective variables).

In particular, in various embodiments, the computer 30 uses for the dataset $200'_1$ (gene expression) and the dataset $200'_2$ (copy number variations) the so-called biweight-midcorrelation metric, since both datasets are of real values. This metrics is *per se* well known and has already been used for determining the correlations between gene expressions. For instance, for this purpose, we may cite the corresponding Wikipedia webpage *"Biweight-midcorrelation"* (available at the link https://en.wikipedia.org/wiki/Biweight_midcorrelation) or the document by Zheng CH, Yuan L, Sha W, Sun ZL, "Gene differential coexpression analysis based on biweight correlation and maximum clique", BMC Bioinformatics, 2014;15 Suppl. 15:S3, doi:10.1186/1471-2105-15-S15-S3, the contents of which are purposely incorporated herein for reference. In general, a Pearson correlation could also be used. However, as already indicated in the article by Zheng, this correlation tends to be too sensitive to outliers.

Instead, in various embodiments, the computer 30 uses for the dataset $200'_3$ (mutations) the mutual information, preferably normalized, since it contains only binary values 0 or 1. Also this measurement of correlation is in itself well known. For instance, for this purpose the corresponding Wikipedia webpage "Mutual information" (available at the link https://en.wikipedia.org/wiki/Mutual_information) may be cited.

Consequently, in step 1104 the computer 30 determines for each dataset $200'_1$, $200'_2$, and $200'_3$ data that identify a measurement of similarity/correlation $s_{ij}$ between the values of each of the p genes of the dataset (i.e., each node $N_i$ that corresponds, respectively, to the variables $p'_1$, $p'_2$, or $p'_3$) and all the other genes of the same dataset (i.e., the other nodes $N_j$ of the network itself). For instance, subsequently these measurements are identified via matrices $S_1$, $S_2$, and $S_3$, respectively, for the networks $202_1$, $202_2$, and $202_3$.

In various embodiments, once the computer 30 has calculated the measurements of similarity/correlation $s_{ij}$, it calculates (in step 1104) the weights $w_{ij}$, for example expressed (as for the matrix S) in the form of a weighted adjacency matrix W of the network, as a function of a respective measurement of similarity/correlation $s_{ij}$. In particular, in various embodiments, the computer 30 converts all the measurements of similarity/correlation $s_{ij}$ into positive values; i.e., the computer 30 determines the absolute value of the measurement of similarity/correlation $s_{ij}$. In various embodiments, the computer 30 then determines a weight $w_{ij}$, moreover scaling the absolute value of the respective measurement $s_{ij}$, preferably by means of an exponential function, for example of the type:

$$w_{ij} = \left| s_{ij} \right|^{\beta} \qquad\qquad (1)$$

This adjacency function (the function that maps the similarity matrix S into an adjacency matrix M) then makes it possible to avoid the disadvantages of a decision via thresholds, i.e., the so-called hard thresholding and, thanks to the use of absolute values, makes it possible to have a contribution for the genes correlated both positively and negatively (e.g., ZHANG, Bin; HORVATH, Steve, "A general framework for weighted gene co-expression network analysis", Statistical applications in genetics and molecular biology, 2005, 4.1). Moreover, the coefficient $\beta$ makes it possible to follow the criterion of the scale-free topology. As will be described in greater detail hereinafter, the structure of the network is subsequently reduced on the basis of the values of the weights $w_{ij}$. Consequently, the value of $\beta$ should be chosen in such a way as to obtain a network with:

- a low density of the nodes N; and/or
- a balanced number of connections between the nodes N of the network.

For instance, in various embodiments the exponent $\beta$ is chosen:

- for the networks $202_1$ (gene expression) and $202_2$ (copy number variations) in the range between 3 and 20, preferably between 5 and 10, for example 6; and
- for the network $202_3$ (mutations) between 1 and 3, for example 1.

Consequently, by the end of step 1104, the computer 30 has determined, for each network $202_1$, $202_2$, and $202_3$, the weights $w_{ij}$ that connect each node/gene N (i.e., the respective variables $p'_1$, $p'_2$ and $p'_3$) to the other nodes/genes N. For instance, subsequently these weights are identified via the matrices $W_1$, $W_2$, and $W_3$ that have been determined, respectively, for the data of the matrices $S_1$, $S_2$, and $S_3$, i.e., respectively, for the networks $202_1$, $202_2$, and $202_3$.

In a step 1106, the computer 30 moreover determines for each gene/node N of a network 200, a measurement of similarity/correlation $s_{ij}$ between the node N and all the nodes N of the other networks, for example between the node $N_1$ of the network $202_1$ and all the nodes $N_i$ of the network $202_2$ and likewise all the nodes $N_i$ of the network $202_3$, for example thus generating:

- a similarity matrix $S_{12}$ between the nodes N of the networks $202_1$ and $202_2$;
- a similarity matrix $S_{13}$ between the nodes N of the networks $202_1$ and $202_3$; and
- a similarity matrix $S_{23}$ between the nodes N of the networks $202_2$ and $202_3$.

In particular, the inventors have noted that the computer can use, for generation of the matrix $S_{12}$ (i.e., of the respective measurements of similarity) between the gene expression and the copy number variations, the biweight-midcorrelation metric. Instead, the computer can use for generation of the other matrices $S_{13}$ and $S_{23}$ the point-biserial correlation. Also this correlation measurement is in itself well known. For instance, for this purpose, the corresponding Wikipedia webpage "Point-biserial correlation coefficient" (available at the link https://en.wikipedia.org/wiki/Point-biserial _correlation_coefficient) may be cited.

Once the computer 30 has determined the measurements of similarity of the nodes between the different networks, for example the matrices $S_{12}$, $S_{13}$, and $S_{23}$, the computer 30 then calculates for each measurement of similarity $s_{ij}$ the respective weight $w_{ij}$. For instance, subsequently these weights are identified via matrices $W_{12}$, $W_{13}$, and $W_{23}$ that have been determined, respectively, for the data of the matrices $S_{12}$, $S_{13}$, and $S_{23}$. For instance, in various embodiments, the computer uses for this purpose again Eq. (1). For instance, in various embodiments, the exponent for the transformation between matrices $S_{12}$, $S_{13}$, and $S_{23}$ and the matrices $W_{12}$, $W_{13}$, and $W_{23}$ is chosen between 2 and 10, preferably between 2 and 5, for example, 4.

Consequently, by the end of step 1104, the computer 30 has determined the weights $W_1$, $W_1$, and $W_2$ that connect the nodes N at the network level, i.e., the intra-network weights, and by the end of step 1106 the weights $W_{12}$, $W_{13}$, and $W_{23}$ that connect the nodes N to the nodes N of the other networks, i.e., the inter-network weights. In general, approximately 50% of the data are redundant since the weight $w_{ij}$ (and likewise the measurement of similarity $s_{ij}$) between the node $N_i$ and the node $N_j$ has the same value as the weight $w_{ji}$ between the node $N_j$ and the node $N_i$. Consequently, these data can be stored also in any other form (for example, in the form of a list), which enables determination of the weight $w_{ij}$ between two nodes $N_i$ and $N_j$, i.e., genes, which may belong to one and the same network or to two different networks.

As explained previously, neglecting the redundancy of the data, each of these weight matrices hence has a size of p $\times$ p values. However, since the number m of reference patients PR is small, the networks obtained in the previous passages are considerably noisy. Consequently, in various embodiments, the computer 30 generates, in a step 1108, for each matrix $W_1$, $W_2$, $W_3$, $W_{12}$, $W_{13}$, and $W_{23}$ a respective matrix W', i.e., matrices $W'_1$, $W'_2$, $W'_3$, $W'_{12}$, $W'_{13}$, and $W'_{23}$, using a backboning procedure configured for extracting the latent structure through pruning/removing of the non-salient edges, i.e., connections between two nodes.

In particular, the inventors have noted that the computer 30 can use in step 1108 the NC (Noise-Corrected) method. The respective method is described in the article by Coscia M., Neffke F. "Network Backboning with Noisy Data", arXiv:1701.07336 [physics.soc-ph]. This method basically consists of three passages:

- initially, the computer transforms the weights $w_{ji}$ of the edges to express them as deviation from their null-model prediction;
- next, the computer calculates the standard deviation for the transformed weights;
- finally, the computer uses the standard deviations to construct the so-called p-values, which are then used to prune/remove the edges.

For instance, the NC method forms part of the software module "Network-Backboning" of Michele Coscia published in Python and available, for example, at https://www.michelecoscia.com/?page_id=287, the contents of which are incorporated herein for this purpose. In general, even though experimental tests have highlighted that NC yields the best result, other backboning methods may also be used, such as other methods supported by the aforementioned software module, e.g.:

- Disparity Filter;
- High Salience Skeleton;
- Doubly Stochastic Transformation;
- Maximum Spanning Tree; or
- Naive Thresholding.

In particular, in various embodiments, the computer 30 prunes, for all the intra-omics networks ($W_1$, $W_2$, and $W_3$) and inter-omics networks ($W_{12}$, $W_{13}$, and $W_{23}$), the branches using a threshold that corresponds to a p-value of 0.05. Consequently, at the end of step 1108, the computer has pruned all the non-salient edges, maintaining only the latent structure of each network $202_1$, $202_2$, $202_3$. For instance, for this purpose the computer can generate matrices $W'_1$, $W'_2$, $W'_3$, $W'_{12}$, $W'_{13}$, and $W'_{23}$, where the values of the non-salient edges/weights of the matrices $W_1$, $W_2$, $W_3$, $W_{12}$, $W_{13}$, and $W_{23}$, have been set at 0.

In various embodiments, the computer then joins, in a step 1110, all the (e.g., six) interaction networks $W'_1$, $W'_2$, $W'_3$, $W'_{12}$, $W'_{13}$, and $W'_{23}$ obtained in step 1108 in a single multi-layer network, where each layer of the network represents an omics (mRNA gene expression, copy number variations, and mutations), and the inter-omics networks connect the different layers.

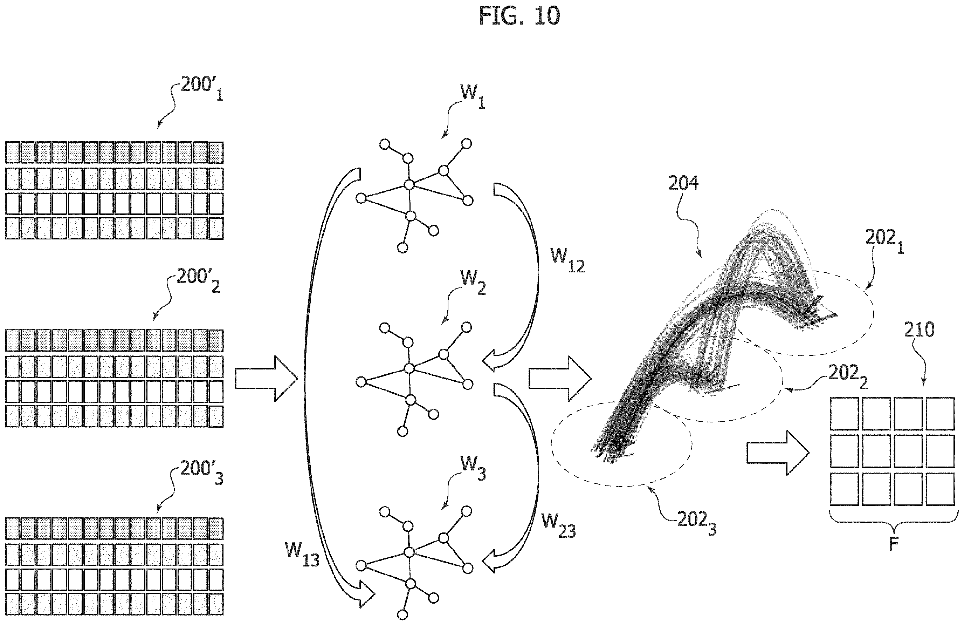

Operation of steps 1104-1110 is also illustrated in Figure 10, where the datasets $200'_1$, $200'_2$, $200'_3$ are used for generating the weights $W_1$, $W_2$, $W_3$, $W_{12}$, $W_{13}$, and $W_{23}$, which in turn are reduced via backboning for generating the multi-layer network 204.

Typically, the resulting network 204 comprises nodes with a reduced number of connections. Consequently, in various embodiments, the computer 30 determines, in a step 1112, sub-networks, the so-called communities, of the multilayer network. For instance, in various embodiments, the computer uses for this purpose the Infomap method in the multilayer version. The Infomap software package is in itself well-known in the technical field and is described, for example, in D. Edler and M. Rosvall, "The MapEquation software package", https://mapequation.github.io/infomap/ (2014-2019). Since the Infomap algorithm is stochastic and iterative, the computer 30 performs a given number of Infomap executions, for example chosen between 20 and 500, for example between 50 and 200, for example 100 executions, and the final structure of the communities is the result at the end of these Infomap executions. Basically, the computer calculates, for each execution/iteration, a measurement of the mean description length per step of a random walker that moves along the (weighted) connections/edges between the nodes of the network. For each iteration, a new partitioning of the network into communities is recorded if the description length is shorter than the previous shortest description length.

In various embodiments, the computer 30 then generates a list of communities 206 by selecting only the communities determined by Infomap that comprise a plurality of nodes. For instance, with reference to the dataset described previously, the computer 30 can extract 217 communities (with a number of nodes) for the p = 15736 genes. In particular, thanks to the use of a multilayer network 204, the communities may comprise nodes of different networks 202, i.e., information on the genes from different omics.

In general, even though experimental tests have highlighted that Infomap yields the best result, other methods for determining the communities/sub-networks may also be used, such as: improved label propagation algorithm (e.g., Li H, Zhang R, Zhao Z, Liu X, "LPA-MNI: An Improved Label Propagation Algorithm Based on Modularity and Node Importance for Community Detection", Entropy (Basel), 2021 April 21, 23(5):497. doi: 10.3390/e23050497); nonnegative matrix factorization (e.g., see Wikipedia webpage "Non-negative matrix factorization"; available at https://en.wikipedia.org/wiki/Non-negative_matrix_factorization), etc.

Once the computer 30 has obtained the list of communities 206, it performs, in a step 1114, a feature-extraction operation, where the computer 30 extracts from each community in the list 206 one or more synthetic variables, where the information of interrelation between the nodes of the communities, i.e., of the data that may refer to different genes and/or different omics, is synthesized.

Consequently, in various embodiments, the computer 30 uses, in step 1112, a method of dimensionality reduction that receives as input the values that each gene has in each omics belonging to the community and returns at output one (or more) synthetic variables, the so-called features F. For instance, in various embodiments, the computer can generate one or more features F using, for this purpose, the UMAP (Uniform Manifold Approximation and Projection) method and the corresponding software package as nonlinear dimensionality-reduction technique.

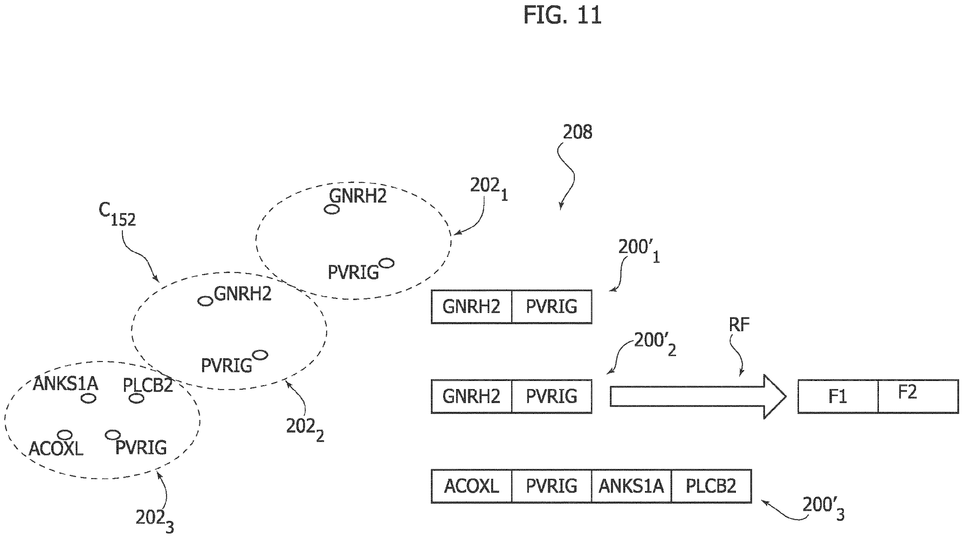

For instance, this is illustrated in Figure 11, where the community $C_{152}$ comprises:

- the mRNA gene expression of the genes GNRH2 and PVRIG;
- the variation in copy numbers of the genes GNRH2 and PVRIG; and
- the information of mutation of the genes ANKS1A, PLCB2, ACOXL, and PVRIG.

In general, even though experimental tests have highlighted that UMAP yields the best result, other methods of feature extraction/dimensional reduction may also be used.

Consequently, the computer generates for each community a dataset 208 that comprises the respective data of the datasets $200'_1$, $200'_2$, and $200'_3$, and uses the feature-extraction algorithm, e.g., UMAP, to generate one or more features F. In particular, in various embodiments, the computer 30 is configured for dividing the variables of a community into a first subset of variables that comprises the variables of the community with continuous values (in particular, the gene expressions $p_1$ and the copy number variations $p_2$) and a second subset of variables that comprises the variables of the community with discrete values (in particular, the mutations $p_3$). In this case, the computer can then generate for each subset of variables a respective dataset 208, which comprises the respective data of the datasets $200'_1$, $200'_2$, and $200'_3$, and use the feature-extraction algorithm to generate one or more features F for the respective subset of variables, for example one or more features F1 for the first subset of variables and one or more features F2 for the second subset of variables.

In the embodiment considered, the computer 30 stores, in step 1114, also the mapping rules RF used for generating each of the features F as a function of the respective variables $p'_1$, $p'_2$, and/or $p'_3$. For instance, assuming that the computer has identified 217 communities, and the variables ($p'_1$, $p'_2$, and/or $p'_3$) that belong to each community are transformed via a respective rule RF into two features F1 and F2, the solution here proposed obtains a total of 434 features F for the 47208 (15736 $\times$ 3) initial variables.

Consequently, in a step 1116, the computer generates a training dataset 210 by using the mapping rules RF for calculating, for each reference patient PR, the values of the features F as a function of the respective data 200 of the reference patient PR (see Figure 12). For instance, the value of the first feature of each reference patient PR is calculated as a function of the data 200 of the respective reference patient PR, using the first mapping rule RF.

Consequently, in a step 1118, the computer can use the training dataset 210 for training a classifier, such as a machine-learning algorithm, which is able to estimate the value of the variable of interest v as a function of the values of a given set of features F. For instance, the classifier may be an artificial neural network, a support-vector machine, or any other parameterized mathematical function configured for estimating the variable of interest v as a function of the values of the features F. In this case, the computer 30 can vary, in step 1118, the values of the parameters PC of the mathematical function in such a way as to reduce a cost function calculated on the basis of the differences between the estimates v' of the variable v supplied by the mathematical function and the value of the variable v in the dataset 210. Finally, the training procedure 1100 terminates in an end step 1120. In this context, the Italian patent application No. 102022000001817, the contents of which are incorporated herein for reference, describes a machine-learning method that is able to estimate the disease-free survival time of a patient. In particular, this document proposes the use of PCA (Principal-Component Analysis) for extraction of features from the omics data. Consequently, the above PCA could be replaced with the solution described herein.

Figure 13 shows an embodiment of the step 1200. In particular, when the step 1200 has been started, the computer 30 obtains the data 300 of the patient P and uses the mapping rules RF for calculating the values 302 of the features F as a function of the data 300 of the patient P (see also Figure 14). Next, the computer 30 uses the trained classifier, as identified, for example, via the values PC of the parameters of the mathematical function, to supply the estimates v' of the variable of interest v as a function of the values 302 of the features F determined for the patient. Finally, the procedure terminates in an end step 1206.

Consequently, the solutions described herein enable considerable reduction of the number of variables/features that must be taken into consideration by the classifier (steps 1118 and 1204). Moreover, irrespective of the specific variable of interest v, the method is able to identify communities of variables that are connected together. Consequently, by analysing, via one or more further feature-extraction algorithms, the link between the variable of interest v and each feature F, the computer 30 can determine the correlation between the variable of interest v and each community (and hence the respective variables $p'_1$, $p'_2$, and/or $p'_3$), which hence enables analysis also of possible biological aspects that can explain the link between given genes and given diseases.

Of course, without prejudice to the underlying principles of the invention, the details of implementation and the embodiments may vary widely with respect to what has been described and illustrated herein purely by way of example, without thereby departing from the scope of the present invention as this is defined in the annexed claims.

**Claims**

1. A method for estimating a variable of interest (v) associated to a given disease as a function of omics data (300) of a patient (P), wherein the method comprises executing the following steps via at least one processor (30):

   - during a training phase (1100):
   - receiving (1102) a first dataset of omics data ($200'_1$) and a second dataset of omics data ($200'_2$), where each dataset of omics data ($200'_1$, $200'_2$) comprises the values of a respective plurality of variables ($p'_1$, $p'_2$) that refer to the same genes for each reference patient (PR) of a plurality of reference patients (PR1, PR2, PR3);
   - generating (1104-1110) a multi-layer network (204) comprising a first layer ($202'_1$) and a second layer ($202'_2$) via the following operations:

      - associating to each variable ($p'_1$) of said first dataset of omics data ($200'_1$) a respective node (N) in said first layer ($202'_1$) and to each variable ($p'_2$) of said second dataset of omics data ($200'_2$) a respective node (N) in said second layer ($202'_2$);
      - generating (1104) intra-omics connections by calculating for each pair of nodes ($N_i$, $N_j$) of the first layer ($202'_1$) and each pair of nodes ($N_i$, $N_j$) of the second layer ($202_2$) a respective similarity value ($s_{ij}$) as a function of the data of the respective variables ($p'_1$, $p'_2$) associated to the two nodes ($N_i$, $N_j$), and calculating, for each pair of nodes ($N_i$, $N_j$) of the first layer ($202'_1$) and each pair of nodes ($N_i$, $N_j$) of the second layer ($202'_2$), a respective weight ($W_1$; $W_2$) associated to the connection between the respective nodes ($N_i$, $N_j$) as a function of the similarity value ($s_{ij}$) of the respective pair of nodes ($N_i$, $N_j$);
      - generating inter-omics connections by calculating for each pair of nodes ($N_i$, $N_j$) between the first layer ($202'_1$) and the second layer ($202'_2$) a respective similarity value ($s_{ij}$) as a function of the data of the respective variables ($p'_1$, $p'_2$) associated to the two nodes ($N_i$, $N_j$), and calculating, for each pair of nodes ($N_i$, $N_j$) between the first layer ($202'_1$) and the second layer ($202'_2$), a respective weight ($W_{12}$) associated to the connection between the respective nodes ($N_i$, $N_j$) as a function of the similarity value ($s_{ij}$) of the respective pair of nodes ($N_i$, $N_j$); and
      - pruning (1108) non-salient intra-omics connections and inter-omics connections of said multi-layer network (204) by applying, to the weights ($W_1$) associated to the intra-omics connections between the nodes of said first layer ($200'_1$), to the weights ($W_2$) associated to the intra-omics connections between the nodes of said second layer ($200'_2$), and to the weights ($W_{12}$) associated to the inter-omics connections between the nodes of said first layer ($202'_1$) and said second layer ($202'_2$), a backboning method;

      - identifying (1112) a plurality of communities (206) of said multi-layer network (204);
      - determining (1114), via a feature-extraction method, for each community (206) one or more respective features (F1, F2) as a function of the values of the variables ($p'_1$, $p'_2$) associated to the nodes (N) that belong to the respective community, and storing the mapping rules (RF) used to generate said one or more features (F1, F2) as a function of the values of the variables ($p'_1$, $p'_2$);
      - generating (1116) a training dataset (210) by obtaining for each reference patient (PR) a respective value of said variable of interest (v) and calculating for each reference patient (PR) the respective values of the features (F1, F2) associated to said communities (206) as a function of the respective values of the variables ($p'_1$, $p'_2$) of the reference patient (PR) by using said mapping rules (RF); and
      - training (1118) a classifier configured for estimating the value (v') of said variable of interest (v) as a function of the values of said features (F1, F2) using said training dataset (210); and
      - during an estimation phase (1200):
      - receiving (1102) the values (300) of the variables ($p'_1$, $p'_2$) of said first dataset of omics data ($200'_1$) and said second dataset of omics data ($200'_2$) for a patient (P);
      - calculating for said patient (P) the values (302) of said features (F1, F2) as a function of the respective values (300) of the variables ($p'_1$, $p'_2$) of the patient (P) using said mapping rules (RF); and
      - estimating (1204) by means of said trained classifier the value (v') of said variable of interest (v) as a function of said values (302) of said features (F1, F2) calculated for said patient (P).

2. The method according to claim 1, comprising receiving (1102) a third dataset of omics data ($200'_3$), wherein said third dataset of omics data ($200'_3$) comprises the values of a respective plurality of variables ($p'_3$) that refer to said genes for said reference patient (PR), and wherein said generating (1104-1110) a multi-layer network (204) comprises:

- associating to each variable (p'$_3$) of said third dataset of omics data (200'$_3$) a respective node (N) in a third layer (202'$_3$);
- generating (1104) intra-omics connections by calculating, for each pair of nodes (N$_i$, N$_j$) of the third layer (202'$_3$), a respective similarity value ($s_{ij}$) as a function of the data of the respective variables (p'$_3$) associated to the two nodes (N$_i$, N$_j$), and calculating for each pair of nodes (N$_i$, N$_j$) of the third layer (202'$_3$) a respective weight (W$_3$) associated to the connection between the respective nodes (N$_i$, N$_j$) as a function of the similarity value ($s_{ij}$) of the respective pair of nodes (N$_i$, N$_j$);
- generating inter-omics connections by calculating, for each pair of nodes (N$_i$, N$_j$) between the first layer (202'$_1$) and the third layer (200'$_3$) and each pair of nodes (N$_i$, N$_j$) between the second layer (202'$_2$) and the third layer (202'$_3$), a respective similarity value ($s_{ij}$) as a function of the data of the respective variables (p'$_1$, p'$_2$, p'$_3$) associated to the two nodes (N$_i$, N$_j$), and calculating, for each pair of nodes (N$_i$, N$_j$) between the first layer (202'$_1$) and the third layer (202'$_3$) and each pair of nodes (N$_i$, N$_j$) between the second layer (202'$_2$) and the third layer (202'$_3$), a respective weight (W$_{13}$, W$_{23}$) associated to the connection between the respective nodes (N$_i$, N$_j$) as a function of the similarity value ($s_{ij}$) of the respective pair of nodes (N$_i$, N$_j$); and
- pruning (1108) the non-salient intra-omics connections and inter-omics connections of said multi-layer network (204) by applying to the weights (W$_3$) associated to the intra-omics connections between the nodes of said third layer (202'$_3$), to the weights (W$_{13}$) associated to the inter-omics connections between the nodes of said first layer (200'$_1$) and said third layer (202'$_3$) and to the weights (W$_{23}$) associated to the inter-omics connections between the nodes of said second layer (202'$_2$) and said third layer (202'$_3$) a backboning method.

3. The method according to claim 1 or claim 2, wherein said first dataset of omics data (200'$_1$), said second dataset of omics data (200'$_2$) and optionally said third dataset of omics data (200'$_3$) are chosen from among:

- a dataset (200'$_1$) of transcriptomic data, where each variable (p'$_1$) corresponds to the gene expression of a particular gene, for example expressed in transcripts per million;
- a dataset (200'$_2$) of copy number variation data, where each variable (p'$_2$) corresponds to the variation of the number of copies of a particular gene; and
- a dataset (200'$_3$) of mutation data, where each variable (p'$_3$) corresponds to the mutation data of a particular gene.

4. The method according to claim 3, wherein said generating (1104) intra-omics connections comprises:

- in the case where the respective dataset of omics data comprises transcriptomic data or copy number variation data, calculating the respective similarity values ($s_{ij}$) via the biweight-midcorrelation metric; and/or
- in the case where the respective dataset of omics data comprises mutation data, calculating the respective similarity values ($s_{ij}$) via the normalized mutual information.

5. The method according to claim 3 or claim 4, wherein said generating (1106) inter-omics connections comprises:

- in the case where the respective dataset of omics data comprise transcriptomic data and copy number variation data, calculating the respective similarity values ($s_{ij}$) via the biweight-midcorrelation metric; and/or
- in the case where the respective dataset of omics data comprise other data, calculating the respective similarity values ($s_{ij}$) via the point-biserial correlation.

6. The method according to any one of the previous claims, wherein said calculating for each pair of nodes (N$_i$, N$_j$) a respective weight $w_{ij}$ associated to the connection between the respective nodes (N$_i$, N$_j$) comprises applying the following equation:

$$w_{ij} = \left|s_{ij}\right|^{\beta}$$

where $s_{ij}$ is the respective similarity value and the exponent $\beta$ is chosen between 1 and 10.

7. The method according to any one of the previous claims, wherein:

- said backboning method is the *Noise-Corrected* method;
- said identifying (1112) a plurality of communities (206) of said multi-layer network (204) comprises performing a plurality of executions of the *Infomap* method; and/or
- said feature-extraction method is the *Uniform-Manifold Approximation-and-Projection* method.

8. The method according to any one of the previous claims, wherein said variable of interest (v) indicates:

   - information whether the respective patient has developed said disease,
   - the severity of said disease which the respective patient has developed, or
   - a disease-free survival time of the respective patient.

9. The method according to any one of the previous claims, wherein said given disease is a neoplasm or cancer, such as a non-small-cell lung cancer.

10. A device (30) configured for implementing the method according to any one of the previous claims.

11. A computer program product that can be loaded into a memory of at least one processor and comprises portions of software code for implementing the steps of the method according to any one of claims 1 to 9.

FIG. 1

FIG. 2

FIG. 3

## FIG. 4

## FIG. 5

## FIG. 6

## FIG. 7

## FIG. 8

FIG. 9

FIG. 10

EP 4 250 301 A1

# FIG. 11

## FIG. 12

F          V

PR1

PR2

PR3

210

## FIG. 13

RF

1200

300

1202          302

V'

1204          PC

1206

## FIG. 14

F

P

302

**EUROPEAN SEARCH REPORT**

| Application Number |
| --- |
| EP 23 16 3839 |

## DOCUMENTS CONSIDERED TO BE RELEVANT

| Category | Citation of document with indication, where appropriate, of relevant passages | Relevant to claim | CLASSIFICATION OF THE APPLICATION (IPC) |
| --- | --- | --- | --- |
| A | TRAN KHOA A. ET AL: "Deep learning in cancer diagnosis, prognosis and treatment selection", GENOME MEDICINE , vol. 13, no. 1 1 December 2021 (2021-12-01), XP055946591, DOI: 10.1186/s13073-021-00968-x Retrieved from the Internet: URL:https://genomemedicine.biomedcentral.com/track/pdf/10.1186/s13073-021-00968-x.pdf * p.3, fig. 2 * ----- | 1-11 | INV. G16B40/20 G16B45/00 G16H50/20 ADD. G16B20/10 G16B20/50 G16B25/10 |
| A | WANI NISAR ET AL: "Integrative approaches to reconstruct regulatory networks from multi-omics data: A review of state-of-the-art methods", COMPUTATIONAL BIOLOGY AND CHEMISTRY, ELSEVIER, AMSTERDAM, NL, vol. 83, 6 September 2019 (2019-09-06), XP085924895, ISSN: 1476-9271, DOI: 10.1016/J.COMPBIOLCHEM.2019.107120 [retrieved on 2019-09-06] * p. 2, 5, 12, 13 and fig.1, 2 and table 1 * ----- -/-- | 1-11 | TECHNICAL FIELDS SEARCHED (IPC) G16B G16H |

The present search report has been drawn up for all claims

| Place of search | Date of completion of the search | Examiner |
| --- | --- | --- |
| Munich | 3 August 2023 | Lüdemann, Susanna |

EPO FORM 1503 03.82 (P04C01)

page 1 of 2

Europäisches Patentamt

European Patent Office

Office européen des brevets

## EUROPEAN SEARCH REPORT

Application Number

EP 23 16 3839

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | ZHENG FAN ET AL: "HiDeF: identifying persistent structures in multiscale 'omics data", GENOME BIOLOGY , vol. 22, no. 1 1 December 2021 (2021-12-01), XP055978601, DOI: 10.1186/s13059-020-02228-4 Retrieved from the Internet: URL:https://genomebiology.biomedcentral.com/counter/pdf/10.1186/s13059-020-02228-4.pdf * title, abstract, p. 10 * | 1-11 | |
| A | MICHELE COSCIA: "The Atlas for the Aspiring Network Scientist", ARXIV.ORG, CORNELL UNIVERSITY LIBRARY, 201 OLIN LIBRARY CORNELL UNIVERSITY ITHACA, NY 14853, 4 January 2021 (2021-01-04), XP081851813, * 24.3, p.343-344, fig. 24.13 * | 1-11 | TECHNICAL FIELDS SEARCHED (IPC) |

The present search report has been drawn up for all claims

| Place of search | Date of completion of the search | Examiner |
|---|---|---|
| Munich | 3 August 2023 | Lüdemann, Susanna |

EPO FORM 1503 03.82 (P04C01)

**EP 4 250 301 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 1977237 B1
- EP 2392678 B1
- EP 2836837 B1
- EP 3237638 B1
- EP 2700038 B1
- IT 102022000001817

### Non-patent literature cited in the description

- **BOCCALETTI, STEFANO et al.** The structure and dynamics of multilayer networks. *Physics reports,* 2014, vol. 544 (1), 1-122
- **KIVELÄ, MIKKO et al.** Multilayer networks. *Journal of complex networks,* 2014, vol. 2 (3), 203-271
- **CANTINI, LAURA et al.** Detection of gene communities in multi-networks reveals cancer drivers. *Scientific reports,* 2015, vol. 5 (1), 1-10
- **HIDALGO, SEBASTIAN J. TERAN ; MA, SHUANGGE.** Clustering multilayer omics data using MuNCut. *BMC genomics,* 2018, vol. 19 (1), 1-13
- **ZHENG CH ; YUAN L ; SHA W ; SUN ZL.** Gene differential coexpression analysis based on biweight correlation and maximum clique. *BMC Bioinformatics,* 2014, vol. 15 (15), S3
- Mutual information. *Wikipedia webpage, https://en.wikipedia.org/wiki/Mutual_information*
- **ZHANG, BIN ; HORVATH, STEVE.** A general framework for weighted gene co-expression network analysis. *Statistical applications in genetics and molecular biology,* 2005, vol. 4, 1
- Point-biserial correlation coefficient. *Wikipedia webpage, https://en.wikipedia.org/wiki/Point-biserial _correlation_coefficient*
- **COSCIA M. ; NEFFKE F.** Network Backboning with Noisy Data. *arXiv:1701.07336*
- **MICHELE COSCIA.** Network-Backboning. *Python*
- **D. EDLER ; M. ROSVALL.** *The MapEquation software package,* 2014, https://mapequation.github.io/infomap/
- **LI H ; ZHANG R ; ZHAO Z ; LIU X.** LPA-MNI: An Improved Label Propagation Algorithm Based on Modularity and Node Importance for Community Detection. *Entropy (Basel),* 21 April 2021, vol. 23 (5), 497
- Non-negative matrix factorization. *Wikipedia webpage, https://en.wikipedia.org/wiki/Non-negative_matrix_factorization*

23